# EUROPEAN PATENT SPECIFICATION

(11) **EP 3 449 968 B1**
(45) Date of publication and mention of the grant of the patent: **17.08.2022**
(21) Application number: 16900580.8
(22) Date of filing: 14.06.2016
(51) Int. Cl.: A61M 27/00, A61M 31/00, A61M 25/00, A61M 25/04, A61B 17/42

(54) **UTERINE CERVIX ADHESION PREVENTION DEVICE**
GEBÄRMUTTERHALSADHÄSIONENSPRÄVENTIONSVORRICHTUNG
DISPOSITIF DE PRÉVENTION DE L'ADHÉSION DU COL DE L'UTÉRUS

(30) Priority: 26.04.2016 KR 20160050984
(43) Date of publication of application: 06.03.2019
(73) Proprietor: Endovision Co., Ltd., Daegu 42709 (KR)
(72) Inventor: JUNG, Min Ho, Daegu 42752 (KR)
(74) Representative: Cabinet Beaumont
(86) International application number: PCT/KR2016/006300
(87) International publication number: WO 2017/188506

(56) References cited:
- DE-A1- 10 112 630
- JP-A- 2004 298 293
- KR-A- 20020 059 287
- KR-A- 20120 020 151
- KR-B1- 101 435 661
- KR-B1- 101 435 661
- KR-B1- 850 000 171
- US-A- 6 027 518
- US-A1- 2013 096 388
- US-B2- 9 226 736

## Description

### Technical Field

The present invention relates to a uterine cervix adhesion prevention device and, more particularly, to a device that is insertable into a uterocervical canal to prevent adhesion and clogging of the uterine cervix after uterine cervix surgery and to help recovery of an affected part.

### Background Art

Female genitalia are composed of ovaries, the uterus, and the vagina, and a part where disease occurs most frequently is the uterus. Meanwhile, the uterus is composed of a uterine body, an endometrium, and a uterocervical canal. More specifically, an entire pathway between the uterus and the vagina is referred to as the uterocervical canal, and the part of the pathway between the uterus and the vagina that is externally visible is often referred to as a uterine cervix. Specially, a part where disease occurs most frequently in the uterus is the uterine cervix.

The uterine cervix is exposed to diseases such as cervical dysplasia, intraepithelial cancer, and invasive cervical cancer due to a direct viral infection by sexual contact, damage, and various stimuli.

When a uterine cervical disease is found by macroscopy or histological biopsy, a cervical conization that excises a cone-shaped portion of the uterine cervix is carried out and the histological changes occurring in this portion are magnified by a microscope and observed for accurate diagnosis. Usually, a recovery period of 4 to 6 weeks is required after cervical conization. After uterine cervical surgery such as the cervical conization, continuous bleeding may occur or inflammation may occur to slow the recovery of damaged uterine cervix. Excessive inflammation may lead to irregular regeneration of damaged tissue, fostering of scarring of regenerated tissue, and excessive tissue regeneration. In addition, after treatment of the uterine cervix, stenosis and clogging of an internal orifice of a uterine cervix may occur, whereby infertility may be induced. Besides infertility, menstrual bleeding may become irregular and the menstrual blood may accumulate in the uterus, thereby causing periodic abdominal pain and menstrual cramps.

When postoperative complications such as adhesion or clogging of the uterine cervix occur as described above, the internal orifice of the uterine cervix is opened or a diameter thereof is widened artificially using a sharp instrument. Although the operation for opening and widening of the uterine cervix is performed, temporary opening thereof cannot be a fundamental solution because the adhesion or clogging of the uterine cervix occurs when the damaged tissue is regenerated. Accordingly, continuous stenosis and clogging symptoms would occur even if surgery and operation for such opening and widening of the uterine cervix were performed.

Accordingly, frequent additional operations are needed, thus causing much suffering and inconvenience to a patient. Consequently, there have been many problems such that the patient suffers from not only great direct pain due the additional operation, since the uterine cervical tissue induces a higher intensity of pain than other parts of a human body, but also a substantial economical burden additionally added thereto.

Conventionally, there was no other way for the patient than to wait for natural recovery or to perform hemostasis, cauterization, and disinfection for the closure and treatment of the surgical site after cervical conization. As a solution to this problem, Korean Patent Registration No. 10-1435661 introduces a tool for recovery after cervical conization, wherein the tool consists of a cervical tube inserted into a uterocervical canal and a thick-walled cap which is integrally formed with or connected to the cervical tube, thereby inducing new tissue to be formed in the same shape as the original uterine cervix by blocking an affected part. However, in the case of Korean Patent Registration No. 10-1435661, since the inserted recovery tool may be displaced or pulled out by the movement of the patient to cause damage to the tissue or delay the recovery, there have been many aspects unsuitable for the tool to function and operate effectively for treating the affected part.

Documents DE 10112630, US 9226736, KR 850000171, US 2013/096388, and US 6027518 disclose devices for helping recovery after surgery. In particular, DE10112630 discloses a uterine cervix adhesion prevention device comprising a hollow drainage tube configured for being inserted into a uterocervical canal, a support cap connected to one end of the hollow drainage tube and configured to extend over a uterus entrance and fixing legs connected to an opposite end of the hollow drainage tube and configured for being unfolded in the uterus to position the hollow drainage tube.

### Disclosure

### Technical Problem

One aspect of the present invention is to provide a uterine cervix adhesion prevention device which prevents uterine cervix adhesion after uterine cervical surgery, is convenient and easy to operate, and can be stably mounted by fixing a position thereof in the uterus.

However, problems to be solved by the present invention are not limited to the above-mentioned problems, and other problems not mentioned will be clearly understood by those skilled in the art from the following description.

### Technical Solution

In order to accomplish the above object, the present invention provides a uterine cervix adhesion prevention device whose one aspect includes: a hollow drainage tube configured for being inserted into a uterocervical canal; a support cap connected to one end of the hollow drainage tube and configured to extend over a uterus entrance (or a uterine cervix); and at least one fixing leg connected to an opposite end of the hollow drainage tube and configured for being unfolded in the uterus to position the hollow drainage tube.

### Advantageous Effects

According to the present invention, it is possible to provide a device capable of preventing uterine cervix adhesions after recovery of the affected part of the uterine cervix by helping the affected part to recover after cervical conization. Meanwhile, the device can be securely mounted so as to avoid a wound of the affected part and can be easily removed, whereby convenience and stability thereof can be improved.

### Description of Drawings

FIG. 1 shows perspective views of a uterine cervix adhesion prevention device according to an embodiment of the present invention.
FIG. 2 shows views illustrating shapes of support caps of a uterine cervix adhesion prevention device according to an embodiment of the present invention.
FIG. 3 shows views illustrating a process of folding a fixing leg by inserting a supporting tube outer holder into a drainage pipe according to a first embodiment of the present invention.
FIG. 4 illustrates a cross-sectional view of a drainage pipe in which a supporting tube outer holder is inserted before a fixing leg is unfolded and a view of a state in which the fixing leg is unfolded, with the supporting tube outer holder being removed.
FIG. 5 illustrates views of shapes of fixing legs and additional legs which are connected to the fixing legs in states before and after, respectively, the supporting tube outer holder is removed according to a first embodiment of the present invention.
FIG. 6 is an exploded perspective view of a uterine cervix adhesion prevention device provided with a supporting tube inner holder according to an example, which does not form part of the present invention.
FIG. 7 illustrates views of states in which a supporting tube inner holder is inserted into a drainage tube, thereby pushing the fixing legs to be unfolded according to the example of Fig.6.
FIG. 8 illustrates a view illustrating a shape where a uterine cervix adhesion prevention device of the present invention is inserted and mounted in a uterus.

### Mode for Invention

Hereinafter, embodiments of the present invention will be described in detail with reference to the accompanying drawings so that those skilled in the art can easily implement the present invention. In order to clearly describe the present invention in the drawings, portions not related to the description have been omitted, and like reference numerals have been assigned to like portions throughout the specification.

Throughout the present specification, when a component is referred to as "comprising" an element, it means that the component may include other elements rather than exclude the other elements unless specifically stated otherwise.

The present invention provides a device for helping the wound area of the cervix heal after cervical surgery by preventing cervical adhesion and clogging.

To this end, a uterine cervix adhesion prevention device of the present invention includes: a hollow drainage tube 120 configured for being inserted into a uterocervical canal; a support cap 110 connected to one end of the hollow drainage tube 120 and configured to be extended over a uterus entrance (or a uterine cervix); and at least one fixing leg 130 connected to the opposite end of the hollow drainage tube 120 and configured for being unfolded in the uterus to position the hollow drainage tube 120.

The uterine cervix adhesion prevention device is preferably made of material harmless to a human body since the uterine cervix adhesion prevention device is held for a recuperation period in a state of being inserted into the body. Accordingly, the uterine cervix adhesion prevention device may be made of silicon, polyethylene, or a composite material containing at least one of the silicon and polyethylene, but is not limited thereto. For example, it will be possible to implement a structure in various forms such that: the hollow drainage tube 120 and the support cap 110 are integrally formed of silicone material, respectively; the hollow drainage tube 120 is formed of silicone material and the support cap 110 is formed of suture material, thereby being connected to each other correspondingly; the hollow drainage tube 120 and the support cap 110 are all made of suture material; or the like.

In the case of the suture material, the suture material is used for suturing the incision site in a process of a general medical surgery or operation and is removed after the treatment of the affected part. However, absorbable material, for example, copolymer such as polylactic acid, polydiaxonone, lactic acid, and glycolic acid may be used, thereby allowing the copolymer to be absorbed in vivo even though the copolymer is not removed after the operation.

The drainage tube 120 is configured for being inserted into the uterocervical canal and is provided with a space formed at the inside thereof. Accordingly, the drainage tube 120 may be a hollow type with a passage 143 through which a secretion generated from the uterus may be discharged to the outside. In addition, the hollow drainage tube 120 may include at least one drainage port 142 for drainage of the secretions on a side thereof.

On the outer circumferential portion of the hollow drainage tube 120, outward projections are integrally formed so as to maintain a state of close connection with the uterus during the recuperation period, thereby maintaining the hollow drainage tube 120 to be in close contact with the inner wall surface of the uterocervical canal.

The support cap 110 may be connected to one end of the hollow drainage tube 120, correspondingly, to extend over a uterus entrance (or a uterine cervix). At this time, the support cap 110 is preferably connected to the hollow drainage tube 120 with the hollow drainage tube 120 as a central axis thereof, correspondingly. That is, one end of the hollow drainage tube 120 is connected to the center of the support cap 110. Meanwhile, the support cap 110 is provided with at least one drainage port 141 on the surface of the support cap 110 so that the support cap 110 can smoothly discharge foreign substances such as inflammatory substances generated while the affected part is recovered, thereby helping the affected part recover.

Even in the case of the drainage port 141 of the support cap 110, when the support cap 110 is made of silicone material, one or a plurality of drainage ports may be integrally formed in a forming process. In the case of suture material, the support cap 110 may be mesh-woven, thereby allowing one or a plurality of drainage ports to be formed naturally.

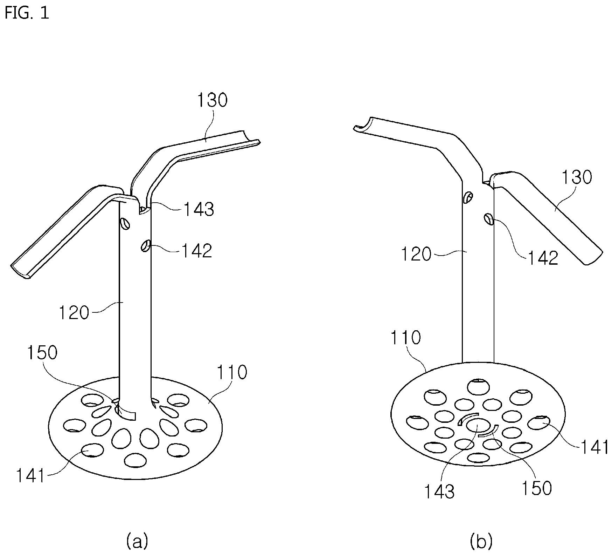

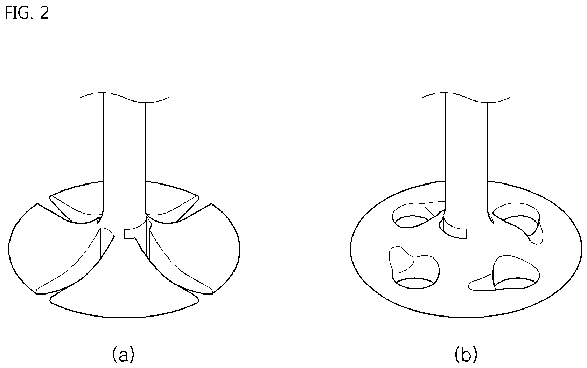

The support cap 110 is a type extending in a predetermined length in all directions around the hollow drainage tube 120 and may be a circular shape, a polygonal shape, a star shape, or a radial shape, but is not limited thereto. Some examples of the shapes of the support cap 110 may be identified through FIG. 2 in addition to FIG. 1.

In addition, since the support cap 110 may be configured to have any shape as long as an overall cross section is a gentle mountain shape or a shape close to the original uterus of the patient, such as a rectangular shape or an elliptical shape, but the shape thereof is not limited. Preferably, the support cap 110 is inclined so that the thickness of the support cap 110 decreases as a distance of the support cap 110 from the hollow drainage tubes 120, correspondingly increases, thereby facilitating smooth drainage.

In addition, the support cap 110 may be formed with projections, or prominences and depressions on the top surface thereof to prevent contact with the uterine cervix.

The fixing leg 130 is connected to an opposite end of the hollow drainage tube 120, and extends in the uterus to position the hollow drainage tube 120 and may be composed of at least one fixing leg 130.

Accordingly, the fixing leg 130 is preferably to be a flexible material.

The fixing leg 130 is formed extending long from the hollow drainage tube 120 in a plurality of directions and includes wing portions each formed extending in the left or right direction on the middle portion thereof, thereby allowing a larger area in the uterus to be secured for fixing the leg 130 therein.

Further, an additional leg 131 may be connected to the distal end of the fixing leg 130 and extends in the longitudinal direction. The additional leg 131 may include wing portions each formed extending in the left or right direction in the middle portion thereof similar to the fixing leg 130.

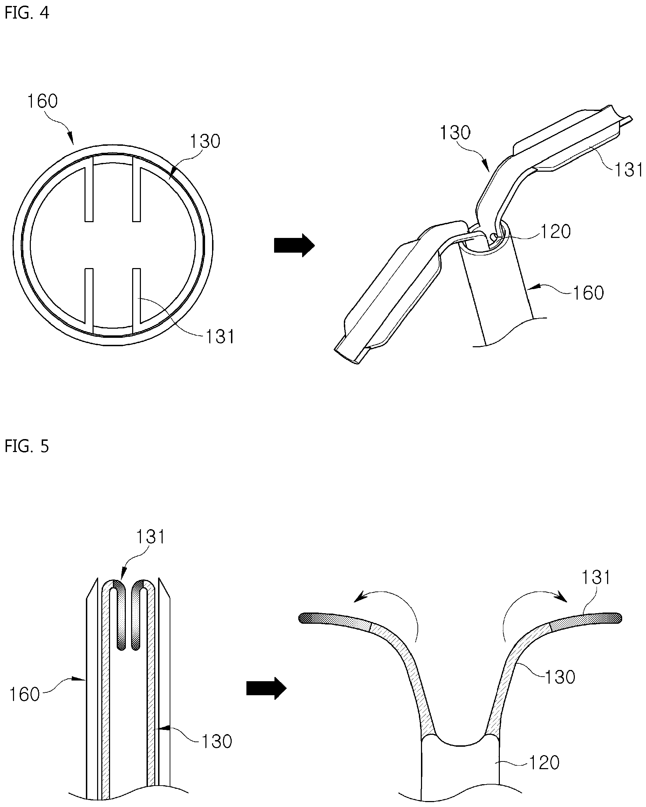

A left drawing of FIG. 4 is a cross-sectional view in which the fixing leg 130 and the additional leg 131 including the wing portions are enclosed inside the supporting tube outer holder 160, and a right drawing of FIG. 4 is a view illustrating a state in which the fixing leg and additional leg are unfolded, with the supporting tube outer holder 160 being removed downwards. The wing portions may be formed of a flexible material together with the fixing leg 130 and the additional leg 131 and may be inserted through the uterocervical canal in a form that is folded inward and enclosed inside the supporting tube outer holder 160.

According to the present invention, an insertion hole 150 is formed at a position where an outer diameter of one end of the hollow drainage tube 120 and the support cap 110 are connected to each other, thereby allowing the supporting tube outer holder 160 to be inserted along the outer diameter portion of the hollow drainage tube 120.

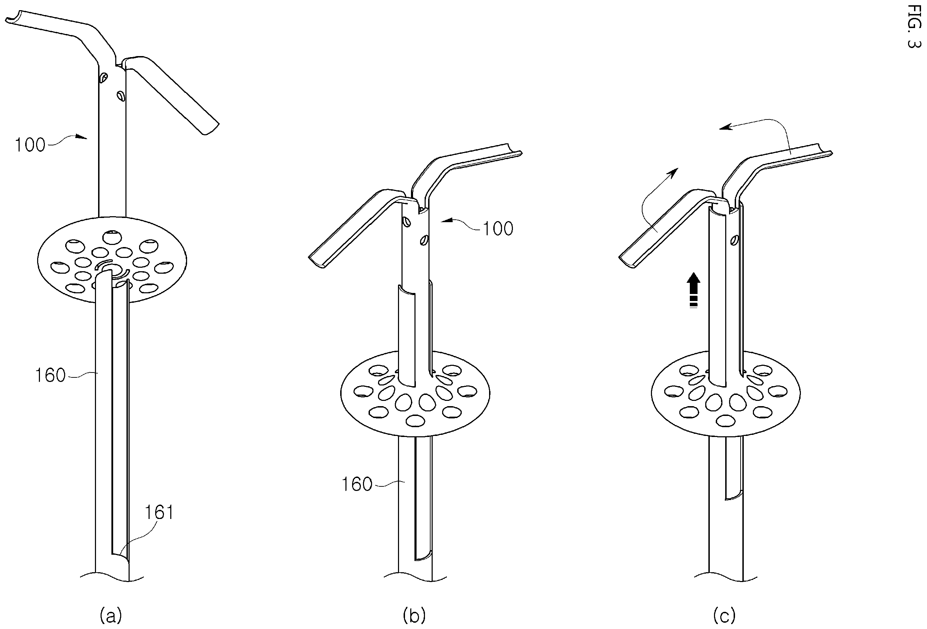

That is, as illustrated in FIG. 3, the uterine cervix adhesion prevention device 100 of the present invention may further be provided with the supporting tube outer holder 160 inserted into the insertion hole 150. Since the supporting tube outer holder 160 is inserted along the outer diameter portion of the hollow drainage tube 120, it can take a part of a tube shape having a diameter larger than that of the hollow drainage tube 120. In addition, the supporting tube outer holder 160 which is to be inserted into the insertion hole 150 may be linearly divided in a lengthwise direction by a length corresponding to the length to be inserted, and thus become a partially opened type.

At this time, the fixing leg 130 is inserted together with the hollow drainage tube 120 through the uterocervical canal by being enclosed by the supporting tube outer holder 160, and then the fixing leg 130 is to be unfolded in the uterus, with the supporting tube outer holder 160 being removed. FIGS. 3a, 3b, and 3c are time-ordered representations of the process of inserting the supporting tube outer holder 160 into the uterine cervix adhesion prevention device 100 of the present invention, and the fixing leg 130 is enclosed inside the supporting tube outer holder 160 as in FIG. 3c.

When the additional leg 131 is connected to and extended from the distal end of the fixing leg 130, as illustrated in FIG. 5, the additional leg 131 which is folded inwards the inside of the fixing leg 130 may be inserted through the uterocervical canal by being enclosed with the supporting tube outer holder 160 and may be unfolded in the uterus together with the fixing leg 130, with the supporting tube outer holder 160 being removed.

In order to prevent the supporting tube outer holder 160 from being inserted too deeply, the supporting tube outer holder 160 may be provided with a stopper 161 for controlling the degree of insertion thereof. The stopper 161 may be composed of a groove, a protrusion, or the like.

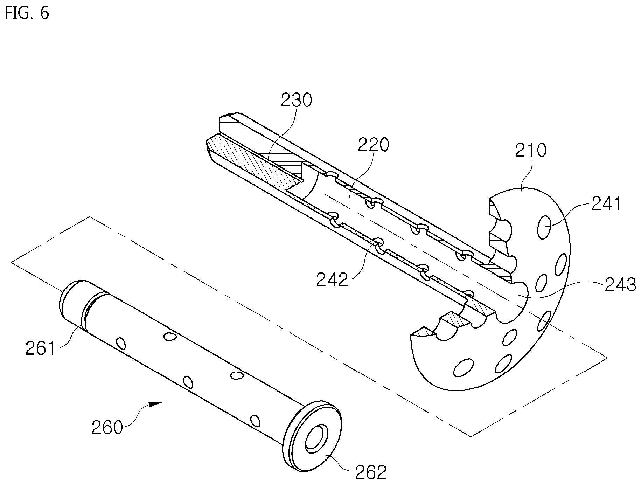

According to an example which does not form part of the present invention, the hollow drainage tube 220 functions as an outer tube and may be additionally provided with a supporting tube inner holder 260 inserted along the inner diameter of the hollow drainage tube 220, as illustrated in FIG. 6.

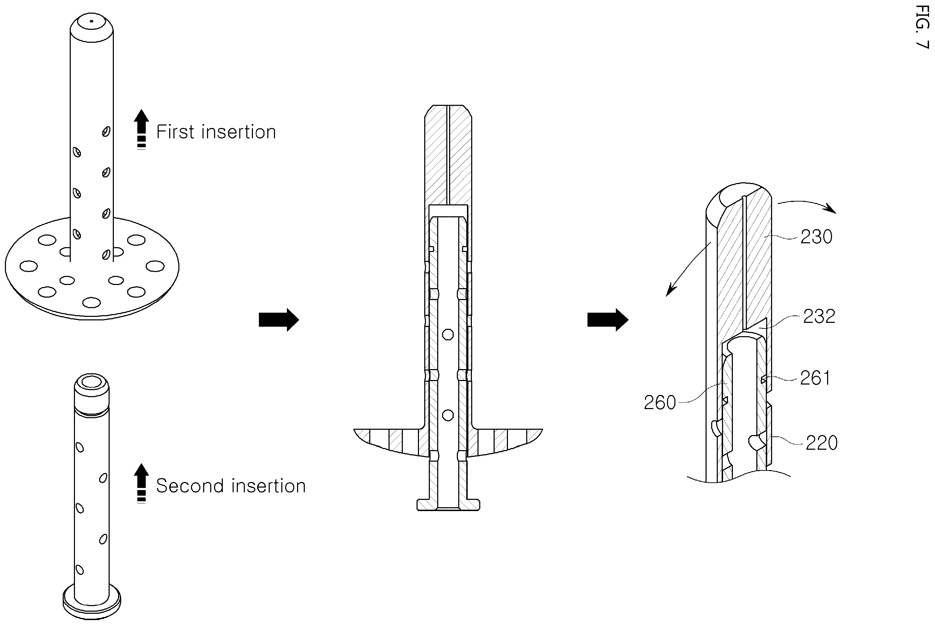

At this time, the fixing leg 230 may be composed to be inserted through the uterocervical canal while being fixed to the opposite end of the hollow drainage tube 220. Subsequently, the supporting tube inner holder 260 inserted along the inner diameter of the hollow drainage tube 220 is configured to push the fixing leg 230 to be unfolded in the uterus. Alternatively, the fixing leg 230 may be configured to be inserted through the uterocervical canal while being disposed inside the hollow drainage tube 220 and to be unfolded into the uterus by the inserted supporting tube inner holder 260 (See FIG. 7.). It may also be applied to the case where the additional leg as described above is connected to and extended from the distal end of the fixing leg 230 (not shown).

In this case, in order to prevent the supporting tube inner holder 260 from being inserted too deeply, the supporting tube inner holder 260 is provided with a fixing groove 261, and the hollow drainage tube 220 is provided with a fixing portion 232, thereby allowing the insertion degree of the supporting tube inner holder to be controlled. At this time, the supporting tube inner holder 260 being inserted into the hollow drainage tube 220 is caught by the fixing portion 232 of the hollow drainage tube 220 and is stopped by the fixing groove 261, thereby allowing the fixing leg 230 to be pushed and unfolded in the process.

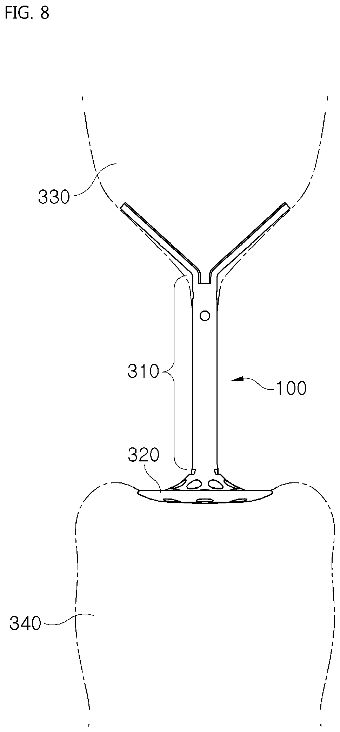

A status of use of the uterine cervix adhesion prevention device of the present invention can be identified through FIG. 8. FIG. 8 is a simplified representation of the appearance of the uterus and illustrates that the uterine cervix adhesion prevention device 100 is inserted into the uterocervical canal 310 located between the vagina 340 and the uterine cavity 330, with the support cap 110 being extended over a uterus entrance 320 and the fixing leg 130 being unfolded to the inside of the uterine cavity 330, thereby holding the position.

After the cervical conization is completed, the uterine cervix adhesion prevention device 100 of the present invention inserted through the uterocervical canal 310 can prevent stenosis or adhesion of the uterine cervix. The secretions generated in the uterus can be discharged through the drainage tube 120 and the drainage port 142 formed on the side of the drainage tube 120. In addition, foreign substances generated during the recovery process of the affected part may be discharged to the outside through at least one above-described drainage port 141 formed on the support cap 110.

Accordingly, it is possible to prevent the affected part from being infected or recover from being slowed down by the foreign substances, thereby helping the affected part quickly recover.

In addition, with the fixing leg 130 provided for positioning the drainage tube 120 in the uterus, it is possible to prevent tissue damage and recovery delays due to the case when the uterine cervix adhesion prevention device 100 falls downwards or is displaced from the original position thereof. Further, the device can be easily and conveniently handled, can be securely mounted to avoid injuring the affected part, and can be smoothly removed after restoration of the affected part because it is a structure fixed in the uterus without any special measures, whereby safety in the use thereof can be improved.

## Claims

1. A uterine cervix adhesion prevention device (100), the device comprising:
a hollow drainage tube (120) configured for being inserted into a uterocervical canal;
a support cap (110) connected to one end of the hollow drainage tube and configured to extend over a uterus entrance; and
at least one fixing leg (130) connected to an opposite end of the hollow drainage tube and configured for being unfolded in the uterus to position the hollow drainage tube,
wherein an insertion hole (150) is formed at a position where an outer diameter of one end of the hollow drainage tube and the support cap are connected to each other, thereby allowing a supporting tube outer holder (160) to be inserted along an outer diameter portion of the hollow drainage tube.

2. The device of claim 1, wherein the hollow drainage tube (120) is provided with at least one drainage port (142) for draining on a lateral surface thereof.

3. The device of claim 1, wherein outward projections are further provided to maintain a state of close connection with an inner wall surface of the uterocervical canal by being formed on an outer circumferential portion of the hollow drainage tube (120).

4. The device of claim 1, wherein the support cap (110) is provided with at least one drainage port (141) for drainage.

5. The device of claim 1, wherein the support cap (110) is a type extending in a predetermined length in all directions with the hollow drainage tube (150) as a central axis and is configured to be a circular shape, a polygonal shape, a star shape, or a radial shape.

6. The device of claim 1, wherein the support cap (110) is inclined so that a thickness thereof decreases as a distance thereof from the hollow drainage tube increases.

7. The device of claim 1, wherein the support cap (110) is formed with projections, or prominence and depression on a top surface thereof for prevention of close contact with a uterine cervix.

8. The device of claim 1, wherein the uterine cervix adhesion prevention device is configured to be further provided with the supporting tube outer holder (160) inserted into the insertion hole (150), and the fixing leg (130) is configured for being inserted together with the hollow drainage tube (120) through the uterocervical canal by being enclosed by the supporting tube outer holder, and then unfolded in the uterus, with the supporting tube outer holder being removed.

9. The device of claim 1, wherein the fixing leg (130) is formed extending long from the hollow drainage tube (120) in a plurality of directions and includes wing portions each formed extending in a left or right direction on the middle portion.

10. The device of claim 1, wherein an additional leg (131) is connected to a distal end of the fixing leg.

11. The device of claim 10, wherein the additional leg (131) includes wing portions each formed extending long from the fixing leg and each formed extending in the left or right direction in the middle portion thereof.

12. The device of claim 1, wherein the supporting tube outer holder (160) is provided with a stopper (161) for controlling the degree of insertion thereof.

## Patentansprüche

1. Vorrichtung (100) zur Verhinderung der Adhäsion des Gebärmutterhalses, wobei die Vorrichtung Folgendes aufweist:
ein hohles Abfluss- bzw. Drainagerohr (120), das konfiguriert ist, um in einen Gebärmutterhalskanal eingeführt zu werden;
eine Stützkappe (110), die mit einem Ende des hohlen Drainagerohres verbunden ist und konfiguriert ist, um sich über einen Eingang in die Gebärmutter zu erstrecken; und
mindestens einen Halte- bzw. Befestigungsschenkel (130), der mit einem gegenüberliegenden Ende des hohlen Drainagerohrs verbunden ist und konfiguriert ist, um in der Gebärmutter entfaltet zu werden, um das hohle Drainagerohr zu positionieren,
wobei ein Einführungsloch (150) an einer Position ausgebildet ist, wo ein Außendurchmesser von einem Endes des hohlen Drainagerohres und die Stützkappe miteinander verbunden sind, wodurch ein äußerer Trag- bzw. Stützrohrhalter (160) entlang eines Abschnittes des äußeren Umfangs des hohlen Drainagerohres eingeführt werden kann.

2. Vorrichtung nach Anspruch 1, wobei das hohle Drainagerohr (120) mit mindestens einer Abfluss- bzw. Drainageöffnung (142) zum Drainieren auf einer Seitenfläche davon versehen ist.

3. Vorrichtung nach Anspruch 1, wobei ferner nach außen gerichtete Vorsprünge vorgesehen sind, um einen Zustand der engen Verbindung mit einer Innenwandfläche des Gebärmutterhalskanals aufrechtzuerhalten, indem sie an einem Abschnitt des äußeren Umfangs des hohlen Drainagerohrs (120) ausgebildet sind.

4. Vorrichtung nach Anspruch 1, wobei die Stützkappe (110) mindestens ein Drainageloch (141) zur Drainage aufweist.

5. Vorrichtung nach Anspruch 1, wobei die Stützkappe (110) von einer Art ist, die sich in einer vorbestimmten Länge mit dem hohlen Drainagerohr (150) als einer Mittelachse in alle Richtungen erstreckt, und die konfiguriert ist, eine kreisförmige Form, eine polygonale Form, eine Sternform oder eine radiale Form zu sein.

6. Vorrichtung nach Anspruch 1, wobei die Stützkappe (110) so geneigt ist, dass ihre Dicke abnimmt, wenn ihr Abstand von dem hohlen Drainagerohr zunimmt.

7. Vorrichtung nach Anspruch 1, wobei die Stützkappe (110) mit Vorsprüngen oder einer Erhebung und Vertiefung auf einer Oberseite davon zur Verhinderung eines engen Kontaktes mit einem Gebärmutter-hals.ausgebildet ist.

8. Vorrichtung nach Anspruch 1, wobei die Vorrichtung zur Verhinderung der Adhäsion des Gebärmutterhalses konfiguriert ist, um ferner mit dem äußeren Stützrohrhalter (160) versehen zu sein, der in das Einführungsloch (150) eingeführt ist, und wobei der Befestigungsschenkel (130) konfiguriert ist, um zusammen mit dem hohlen Drainagerohr (120) durch den Gebärmutterhalskanal eingeführt zu werden, indem er von dem äußeren Stützrohrhalter umschlossen wird, und dann in der Gebärmutter entfaltet wird, wobei der äußere Stützrohrhalter entfernt wird.

9. Vorrichtung nach Anspruch 1, wobei der Befestigungsschenkel (130) so ausgebildet ist, dass er sich lang von dem hohlen Drainagerohr (120) in eine Vielzahl von Richtungen erstreckt und Flügelabschnitte aufweist, wobei jeder so geformt ist, dass er sich in einer linken oder rechten Richtung auf dem mittleren Abschnitt erstreckt.

10. Vorrichtung nach Anspruch 1, wobei ein zusätzlicher Schenkel (131) mit einem distalen Ende des Befestigungsschenkels verbunden ist.

11. Vorrichtung nach Anspruch 10, wobei der zusätzliche Schenkel (131) Flügelabschnitte aufweist, wobei jeder so geformt ist, dass er sich lang von dem Befestigungsschenkel erstreckt, und jeder so geformt ist, dass er sich in der linken oder rechten Richtung in dem mittleren Abschnitt davon erstreckt.

12. Vorrichtung nach Anspruch 1, wobei der äußere Stützrohrhalter (160) mit einem Stopper (161) versehen ist, um das Ausmaß seiner Einführung zu steuern.

## Revendications

1. Dispositif de prévention de l'adhérence du col de l'utérus (100), le dispositif comprenant :
un tube de drainage creux (120) configuré pour être inséré dans un canal utéro-cervical ;
un capuchon de support (110) connecté à une extrémité du tube de drainage creux et configuré pour s'étendre sur une entrée de l'utérus ; et
au moins une patte de fixation (130) connectée à une extrémité opposée du tube de drainage creux et configurée pour être dépliée dans l'utérus pour positionner le tube de drainage creux,
dans lequel un orifice d'insertion (150) est formé à une position où un diamètre externe d'une extrémité du tube de drainage creux et le capuchon de support sont connectés l'un à l'autre, permettant ainsi à un support externe de tube de support (160) d'être inséré le long d'une partie de diamètre externe du tube de drainage creux.

2. Dispositif selon la revendication 1, dans lequel le tube de drainage creux (120) est muni d'au moins un orifice de drainage (142) pour le drainage sur une de ses surfaces latérales.

3. Dispositif selon la revendication 1, dans lequel des saillies vers l'extérieur sont en outre prévues pour maintenir un état de connexion étroite avec une surface de paroi interne du canal utéro-cervical en étant formées sur une partie circonférentielle externe du tube de drainage creux (120).

4. Dispositif selon la revendication 1, dans lequel le capuchon de support (110) est muni d'au moins un orifice de drainage (141) pour le drainage.

5. Dispositif selon la revendication 1, dans lequel le capuchon de support (110) est d'un type s'étendant d'une longueur prédéterminée dans toutes les directions avec le tube de drainage creux (150) comme axe central et est configuré pour avoir une forme circulaire, une forme polygonale, une forme en étoile ou une forme radiale.

6. Dispositif selon la revendication 1, dans lequel le capuchon de support (110) est incliné de sorte que son épaisseur diminue à mesure que sa distance par rapport au tube de drainage creux augmente.

7. Dispositif selon la revendication 1, dans lequel le capuchon de support (110) est formé avec des saillies, ou une proéminence et une dépression sur sa surface supérieure pour empêcher un contact étroit avec un col de l'utérus.

8. Dispositif selon la revendication 1, dans lequel le dispositif de prévention de l'adhérence du col de l'utérus est configuré pour être en outre muni du support externe de tube de support (160) inséré dans l'orifice d'insertion (150), et la patte de fixation (130) est configurée pour être insérée avec le tube de drainage creux (120) à travers le canal utéro-cervical en étant enfermée par le support extérieur du tube de support, puis dépliée dans l'utérus, pendant le retrait du support extérieur du tube de support.

9. Dispositif selon la revendication 1, dans lequel la patte de fixation (130) est formée s'étendant loin du tube de drainage creux (120) dans une pluralité de directions et comporte des parties d'aile formées chacune s'étendant dans une direction gauche ou droite sur la partie médiane.

10. Dispositif selon la revendication 1, dans lequel une patte supplémentaire (131) est connectée à une extrémité distale de la patte de fixation.

11. Dispositif selon la revendication 10, dans lequel la patte supplémentaire (131) comporte des parties d'aile formées chacune s'étendant loin de la patte de fixation et formées chacune s'étendant dans la direction gauche ou droite dans sa partie médiane.

12. Dispositif selon la revendication 1, dans lequel le support externe de tube de support (160) est muni d'un bouchon (161) pour contrôler son degré d'insertion.
